# EUROPEAN PATENT APPLICATION

(11) **EP 1 921 087 A1**
(43) Date of publication of application: **14.05.2008**
(21) Application number: 06023423.4
(22) Date of filing: 10.11.2006
(51) Int. Cl.: C07K 1/06, C07K 7/56, C07K 14/48

(54) **Process for the preparation of cyclic peptides**

(71) Applicant: PRIMM S.R.L., 20132 Milano (IT)
(72) Inventor: Sanseverino, Marina, 80131 Napoli (IT); Astarita, Daniela, 80135 Napoli (IT)
(74) Representative: Minoja, Fabrizio

(57) **Abstract**

The present invention relates to the synthesis and purification of NGF-like peptides of formula (I): 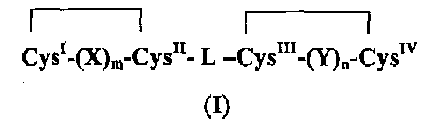 wherein X, Y m, n and L are as defined in the description the process comprising:
a) synthesising a linear peptide of formula (II):

**Cys^{I}(P)-(X)ₘ(P)-Cys^{II}(P)- L(P) -Cys^{III}(P)- (Y)ₙ(P) -Cys^{IV}(P)** **(II)**

wherein P represent a protecting group, with the proviso that the P groups on Cys^{I} and Cys^{II} are TFA-labile protecting groups and the P groups on Cys^{III} and Cys^{IV} are TFA-stable protecting groups;
b) selectively removing the TFA-labile protecting groups with contemporary release of the linear peptide from resin;
c) dissolving the resulting peptide in diluted phosphate buffer and exposing the solution to atmospheric oxygen so as to form a disulfide bridge between Cys^{I} and Cys^{II};
d) purifying the resulting peptide by HPLC;
e) dissolving the peptide in TFA/anisole containing thallium trifluoroacetate so as to form a disulfide bridge between Cys^{III} and Cys^{IV};
f) submitting the resulting peptide to gel filtration.

SEQUENCE LISTING

<110>  PRIMM S.R.L.

<120>  PROCESS FOR THE PREPARATION OF CYCLIC PEPTIDES

<130>  1751eur

<160>  3

<170>  PatentIn version 3.3

<210>  1
<211>  17
<212>  PRT
<213>  Unknown

<220>
<223>  SYNTHETIC PEPTIDE

<220>
<221>  DISULFID
<222>  (1)..(8)

<220>
<221>  DISULFID
<222>  (12)..(17)

<400>  1

Cys Thr Asp Ile Lys Gly Lys Cys Thr Gly Ala Cys Asp Gly Lys Gln
1               5                   10                  15

Cys

<210>  2
<211>  17
<212>  PRT
<213>  Unknown

<220>
<223>  SYNTHETIC PEPTIDE

<220>
<221>  DISULFID
<222>  (1)..(8)

<220>
<221>  DISULFID
<222>  (12)..(17)

<400>  2

Cys Thr Asp Ile Arg Gly Asn Cys Thr Gly Ala Cys Asp Gly Lys Gln
1               5                   10                  15

Cys

<210>  3
<211>  17
<212>  PRT
<213>  Unknown

<220>
<223>  SYNTHETIC PEPTIDE


<220>
<221>  DISULFID
<222>  (1)..(8)

<220>
<221>  DISULFID
<222>  (12)..(17)

<400>  3

Cys Thr Asp Ile Lys Gly Lys Cys Thr Gly Ala Cys Asp Asp Arg Gln
1               5                   10                  15


Cys

## Description

### Field of the invention

The present invention relates to the synthesis and purification of peptides containing disulfide bridges, in particular of NGF-like peptides containing two disulfide bridges.

### Background of the invention

Spontaneous formation of multiple disulfide bridges in peptides with correct pairing of cysteine residues can be observed in naturally occurring peptides, but it is very difficult to achieve in synthetic peptides.

The preparation of synthetic peptides is disclosed, for example, in: Fmoc Solid Phase Peptide Synthesis, A Practical Approach Edited by W. C. Chan and P. D. White, 2000 - Synthetic Peptides A User's Guide (2002 second edition) Gregory A. Grant, editor - Oxford University Press, New York - Solid-Phase Synthesis A Practical Guide (2000) Editors, Steven A. Kates and Fernando Albericio. The cyclization of synthetic peptides is usually carried out in a dilute solution of a free peptide by air oxidation, which often leads to a complex mixture of cyclic monomers and linear polymers. As a consequence, isolation and purification of the desired product is troublesome and often requires HPLC.

Better results can be achieved with multi-step processes (two step processes in the case of peptides containing only two disulfide bridges), which comprise protecting the cysteine residues with different protective groups and forming the disulfide bridges in sequence, by selectively removing the protective groups. Such multi-step processes can be carried out either on fully protected peptides bound to a solid support or on partially protected peptides in solution. However, there is still the need to establish which disulfide bridge must be formed first, because the structural and/or conformational local hindrance of each disulfide bridge may affect the formation of other disulfide bridges and therefore the overall yield of the process.

Therefore, it would be desirable to provide a process for the preparation of cyclic peptides containing disulfide bridges which is free from the above-mentioned drawbacks.

### Description of the invention

The present invention relates to a process for the synthesis and purification of cyclic peptides containing two disulfide bridges of formula (I): 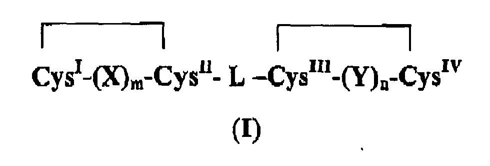 in which:
X and Y, independently from one another, represent each an amino acid;
m and n, independently from one another, represent each an integer ranging from 4 to 8;
L is a linker formed by 2 to 4 amino acids, or an organic linker
the process comprising the following steps:
a) synthesising a linear peptide of formula (II):

   **Cys^{I}(P)-(X)ₘ(P)-Cys^{II}(P)- L(P) -Cys^{III}(P)- (Y)ₙ(P) -Cys^{IV}(P)** (II)

   wherein X, Y, m, n and L are as defined above and P represent protecting groups conventionally used in peptide synthesis, with the proviso that the P groups on Cys^{I} and Cys^{II} are TFA-labile protecting groups and the P groups on Cys^{III} and Cys^{IV} are TFA-stable protecting groups;
b) selectively removing the TFA-labile protecting groups with contemporary release of the linear peptide from resin;
c) dissolving the resulting linear peptide in diluted phosphate buffer at pH 7-8 and exposing the solution to atmospheric oxygen so as to form a disulfide bridge between Cys^{I} and Cys^{II} ;
d) purifying the resulting peptide by HPLC;
e) dissolving the peptide at a concentration of 0.05 - 0.15 mM in a TFA/anisole solution containing thallium trifluoroacetate so as to form a disulfide bridge between Cys^{III} and Cys^{IV};
f) submitting the resulting peptide to gel filtration.

In the compounds of formula (I) (X)ₘ is an amino acid sequence preferably selected from TDIKGK, i.e. residues 29-38 of NGF, and TDIRGN; (Y)ₙ sequence is an amino acid sequence preferably selected from DGKQ, i.e. residues 92 - 97 of NGF, and DDRQ.

The organic linkers L are amino carboxylic acids containing a number of methylene (-CH₂-) units ranging from 2 to 11, and preferably 7, or polyoxoethylene amino carboxylic acids containing a number of oxoethylene (-CH₇-CH₂-O-) units from 2 to 4, and preferably 2. Examples of amino carboxylic acids are beta-alanine, 5-aminopentanoic acid, 8-aminooctanoic acid, 11-aminoundecanoic acid; examples of polyoxoethylene amino carboxylic acids are 8-amino-3,6-dioxaoctanoic acid and 12-amino-4,7,10-trioxadodecanoic acid. Preferred L linker is the TGA amino acid sequence. According to a more preferred embodiment, the invention relates to a process for the preparation of the following NGF-like peptides:

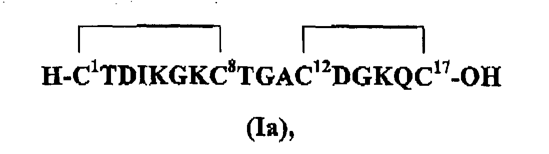

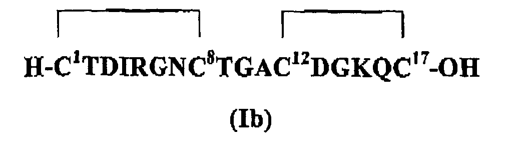

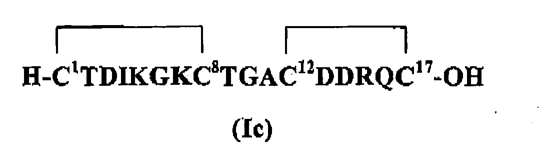

In the following, peptide (Ia) will be referred to as L1L4, peptide (Ib) will be referred to as RNL1-L4 and peptide (Ic) will be referred to as L1-DRL4. In these peptides, the disulfide bridges between the cysteines at positions 1 and 8 and the cysteines at positions 12 and 17 will be also referred to as loop 1 and loop 4 respectively.

The synthesis of the peptide of formula (**II**) is usually carried out in solid phase using a chlorotrityl resin, which has the advantage of avoiding cysteine racemization when cysteine is attached.

For the purposes of the present invention, the expression "TFA-labile" means removable by treatment with 80-95% TFA in water, with or without scavengers; the expression "TFA-stable" means not removable by treatment with 80-95% TFA in water, with or without scavengers. Examples of TFA-labile protecting groups are trityl (Trt); examples of TFA-stable protecting groups are tBu, which can be removed by treatment with MeSiGl3/PhSOPh and tButhio, which can be removed by treatment with RSH and acetamidine, which can be removed with I₂.

According to a preferred embodiment of the invention, the TFA-labile protecting group on Cys^{I} and Cys^{II} is trityl and the TFA-stable protecting group on Cys^{III} and Cys^{IV} is acetamidine (Acm).

The linear precursor is cleaved off the resin by treatment with a TFA solution, preferably a TFA/water/phenol/thioanisole/ethandithiol/triisopropylsilane solution so as to remove all protecting groups with the exception of the TFA-stable protecting groups. The resulting peptide is purified by preparative HPLC and dissolved in diluted phosphate buffer, so as to oxidize the first cysteine pair. Usually, the solution is allowed to stand for a time comprised between 20 and 30 hours, preferably for about 24 hours, monitoring the reaction by HPLC. When the reaction is complete; the peptide is purified by preparative HPLC, isolated and dissolved in a TFA/anisole solution, typically a 0.1 mM solution of TFA containing thallium trifluoroacetate, and allowed to stand for 0.5 - 2 hours, preferably for about one hour, so as to accomplish removal of the protective groups and oxidation of the second cysteine pair. The reaction is monitored by HPLC. When the reaction is complete, the product is recovered by gel filtration.

The process of the invention has the advantage that the cyclization of the second cysteine pair occurs with quantitative yield and that HPLC purification of the final product is not required; the overall yield is 25-40 times higher than that of known processes.

The invention will be now illustrated in greater detail in the following experimental section.

### EXPERIMENTAL SECTION

A preliminary set of experiments was carried out to find out:
1) the best conditions for the synthesis of linear peptides on a solid support (resin);
2) the most suitable protecting groups on the cysteine residues for selective cyclization;
3) which disulfide bridge must be formed first;
4) whether the synthesis of the cyclic peptides had to be carried out on a resin or in solution.

### Synthesis of linear precursors

The synthesis of linear precursors was carried out on an acid labile chlorotrityl resin using (9H-fluoren-9-ylmethoxy)carbonyl (FMOC) and t-butyl (tBu) as protecting groups and trifluoroacetic acid (TFA) for the final cleavage/deprotection stage. With respect to fluoridric acid (HF) used to remove t-butoxycarbonyl (Boc) and benzyl protecting groups, TFA is much safer, easier to dispose of, and less aggressive towards acid-labile amino acids such as cysteine.

Chlorotrityl resins, which do not require activation of the first amino acid residue, was chosen in order to avoid cysteine racemisation, which occurs when cysteine is activated to bind to hydroxy-derivatized solid supports. To evaluate possible drawbacks, before synthesising the peptides, an *in silico* analysis of the sequence was performed using different programs based on algorithms describing the different physical and chemical properties of the peptide concerned (aggregation potential, spatial accessibility, secondary structure).

A first synthesis on a 30 micromoles scale was performed following standard protocols and with all four cysteines residues protected with a trityl (Trt) group; a double coupling protocol was followed in the next series of syntheses.

### Selection of orthogonal protecting groups for cysteine pairing

The following syntheses were aimed to define the best pair of cysteine-protecting groups for in-sequence formation of the two disulfide bridges on a resin or in solution. The two following sets of protected analogues of the linear precursor of L1L4 were synthesized, wherein:
(X)ₘ(P) is Thr(tBu)-Asp(OtBu)-Ile-Lys(Boc)-Gly-Lys(Boc)
L(P) is Thr(tBu)-Gly-Ala
(Y)ₙ(P) is Asp(OtBu)-Gly-Lys(Boc)-Gln(Trt)

### SET 1 (oxidation in solution)*

1) Fmoc-Cys(Trt)-(X)ₘ(P)-Cys(Trt)-L(P)-Cys(Trt)-(Y)ₙ(P)-Cys(Trt)-resin
2) Fmoc-Cys(tBu)-(X)ₘ(P)-Cys(tBu)-L(P)-Cys(Trt)-(Y),(P)-Cys(Trt)-resin
3) Fmoc-Cys(Trt)-Xaa-Cys(Trt)-L(P)-Cys(tBu)-(Y)ₐ(P)-Cys(tBu)-resin
4) Fmoc-Cys(tButhio)-(X)ₘ(P)-Cys(tButhio)-L(P)-Cys(Trt)-(Y)ₙ(P)-Cys(Trt)-resin
5) Fmoc-Cys(Trt)-(X)ₘ(P)-Cys(Trt)-L(P)-Cys(tButhio)-(Y)ₙP)-Cys(tButhio)-resin
6) Fmoc-Cys(Acm)-(X)ₘ(P)-Cys(Acm)-L(P)-Cys(Trt)-(Y)ₙ(P)-Cys(Trt)-resin
7) Fmoc-Cys (Trt)-(X)ₘ(P)-Cys(Trt)-L(P)-Cys(Acm)-(Y)ₙ(P)-Cys(Acm)-resin

### SET 2 (oxidation on solid support)*

8) Fmoc-Cys(tBu)-(X)ₘ(P)-Cys(tBu)-L(P)-Cys(tButhio)-(Y)ₙ(P)-Cys(tButhio)-resin
9) Fmoc-Cys(tButhio)-(X)ₘ(P)-Cys(tButhio)-L(P)-Cys(tBu)-(Y)ₙ(P)-Cys(tBu)-resin
10) Fmoc-Cys(tBut)-(X)ₘ(P)-Cys(tBu)-L(P)-Cys(Acm)-(Y)ₙ(P)-Cys(Acm)-resin
11) Fmoc-Cys(Acm)-(X)ₘ(P)-Cys(Acm)-L(P)-Cys(tBu)-(Y)ₙ(P)-Cys(tBu)-resin
12) Fmoc-Cys(tButhio)-(X)ₘ(P)-Cys(tButhio)-L(P)-Cys(Acm)-(Y)ₙ(P)-Cys(Acm)-resin
13) Fmoc-Cys(Acm)-(X)ₘ(P)-Cys(Acm)-L(P)-Cys(tButhio)-(Y)ₙ(P)-Cys(tButhio)-resin

* resin substitution 0.3 - 0.5 mmmol/g;
* * resin substitution <0.2 mmmol/g to promote intra-molecular vs intermolecular reactions.

### Results from SET 1

### Analogue 1)

All protecting groups were removed under standard acidic conditions used for peptide cleavage from solid support. Cyclization was performed by air oxidation in dilute solution on the fully deprotected peptide, in order to evaluate the thermodynamic and kinetic ability of the peptide to fold correctly and form selective disulfide bridges. As expected, a very complex mixture of cyclic monomers and polymers was obtained and purifying the desired product was troublesome.

### Analogues 2) and 3)

These analogues were prepared to evaluate the efficiency of the orthogonal pair Trt/tBu and the order of disulfide bridge formation.

All protecting groups were removed under standard acidic conditions used for peptide cleavage from solid supports, except for the tBu protecting group, which was selectively deprotected with MeSiCl₃/PhSOPh. In both cases yields were very low; however, the yields were higher when loop I was formed before loop 4.

### Analogues 4) and 5)

These analogues were prepared to evaluate the efficiency of the orthogonal pair Trt/tButhio and the order of disulfide bridge formation.

All protecting groups were removed under the standard acidic conditions, used for peptide cleavage from solid supports, except for tButhio, which was selectively removed with RSH.

In both cases yields were very low; however, the yields were higher when loop 1 was formed before loop 4.

### Analogues 6) and 7)

These analogues were prepared to evaluate the efficiency of the orthogonal pair Trt/Acm (acetamidine) and the order of disulfide bridge formation.

All protecting groups were removed under the standard acidic conditions used for peptide cleavage from solid supports, except for the Acm group, which was selectively removed with I₂.

Also in this case yields were higher when loop 1 was formed before loop 4 and were also higher than those of analogues 1-5.

### Results from SET 2

Analogues 8) - 13) were prepared to evaluate the efficiency of the orthogonal pairs of the following protecting groups tBu/tButhio, tBu/Acm, tButhio/Acm, and the order of disulfide bridge formation on resins.

Notwithstanding the different deprotection procedures and oxidation conditions, in all cases when formation of loop 1 was tried first, the reaction was incomplete, and loop 4 never formed.

In summary, attempts to selectively oxidize cysteine on solid a support gave poor results. On the contrary, oxidation carried out in solution gave satisfactory results, especially when loop L1 was formed before loop 4.

In particular, the best results were obtained when selective oxidation was performed in solution with cysteine groups protected with Trt/Acm and L 1 was formed first (analogue 7), as described below.

### Synthesis of L1L4 (Ia)

### Solid phase synthesis of the linear precursor

The linear precursor of L1L4 (Ia) was synthesized using Fmoc solid phase strategy starting from a Fmoc-Cys(Acm)-chlorotrityl resin (substitution 0.4 mmol/g), to avoid cysteine racemization. In order to achieve selective formation of the two disulphide bridges (Cys¹/Cys⁸ and Cys¹²/Cys¹⁷), Cys¹ and Cys⁸ were protected with trityl (a TFA-labile group) and Cys¹² and Cys¹⁷ were protected with acetamidine (an iodine-labile protecting group).

The synthesis of the linear precursor was carried out through a series of repeated deprotection/coupling steps. Removal of the Fmoc group was performed with 20% piperidine in DMF. The coupling reactions were performed through activation of the Fmoc-amino acid derivatives with HBTU O-(benzotriazol-1-yl)-N,N,N',N'-tetramethyl-uronium hexafluorophosphate, 4 equivalents) and DIEA (diisopropylethylamine, 8 equivalents) in DMF. Each coupling was repeated twice and monitored with the Kaiser test.

The peptide was cleaved off the resin with contemporary deprotection of all side chain protecting groups, except for the Acm groups on Cys¹² and Cys¹⁷, using a mixture of TFA/H₂O/Phenol/Thioanisole/ethandithiol/triisopropylsilane (81.5:5:5:5:2.5:1 ν/ν/ν/ν/ν/ν). The resin was then filtered off and the linear precursor of L1L4 was precipitated from ethyl ether.

The crude product, with free thiol functions on Cys¹ and Cys⁸ and with Cys¹² and Cys¹⁷ still protected with Acm was purified by preparative HPLC on a SHIMADZU LC system equipped with a UV detector. Elution was performed with a binary system (Eluent A: 0.1% TFA in water; Eluent B: 0,1% TFA in acetonitrile) using a Vydac C₁₈ column (22 x 250 mm; 5µm; 300 A) and a linear gradient from 5% B to 65% B in 40 min at a flow rate of 10 ml/min.

The structure of the purified peptide was confirmed with matrix-assisted laser desorption ionization mass spectrometry (MALDI-MS) on a MALDI-TOF Voyager-DE (Perseptive Biosystem) spectrometer. The MALDI-tof analysis revealed a molecular ion peak [M-H⁺] of 1874 as expected for the linear precursor. Analytical HPLC of the purified peptide on a SHIMADZU LC-10 system equipped with a Diode Array detector showed a purity higher than 95%. Elution was performed with a binary system (Eluent A: 0.1% TFA in water; Eluent B: 0.1% TFA in acetonitrile) using a Vydac C₁₈ column (4.6 x 150 mm; 5 µm; 300 A) and a linear gradient from 5% B to 65% B in 20 min at a flow rate of 1 ml/min.

### Selective disulphide bridge formation in solution

Oxidation of Cys¹/Cys⁸ was carried out in the presence of atmospheric oxygen, at high diluition (0. 1 mM) in phosphate buffer at pH 7-8 for 24 h. The reaction course was monitored by HPLC and the cyclised product was purified by preparative HPLC under the same conditions used for the linear precursor (see above) to a final purity of 80%.

Deprotection and oxidation of Cys¹²/Cys¹⁷ was performed by iodine-mediated oxidation dissolving the peptide at a concentration of 0.1 mM in Ac-OH/H₂O (1:1) in the presence of iodine (10 equivalents) for 30 min., following the reaction by HPLC. After 30 minutes side-product started to form and the reaction was stopped, despite the fact that a considerable percentage of linear precursor was still unreacted. The iodine excess was quenched with sodium thiosulphate.

The peptide containing two disulphide bridges was purified by preparative HPLC under the same conditions as the previous two steps (see above) and identified by MALDI-Tof spectrometry, which gave the expected molecular ion peak [M-H⁺] of 1730 Da,

Analytical HPLC revealed a purity of 90%.

Peptides RNL1-L4 (Ib) and L1-DRL4 (Ic) were prepared in a similar way.

Yields and purity of the three peptides obtained following the above described procedures are reported in Table 1.

The results of the above experiments were considered quite satisfactory for laboratory scale but still poorly adequate for the scaling up on an industrial scale.

Table 1 and Examples 1 - 5 and show that the process of the invention is suitable for industrial scale, as it allows to obtain the cyclic peptides with high purity and yield.

**TABLE 1 - Peptide yields and purities during the synthetic procedures.**

| **Peptide** | **L1L4** | **RNL1-L4** | **L1-DRL4** | **L1L4** | **RNL1-L4** | **L1-DRL4** | **L1L4** | **L1L4** |
|---|---|---|---|---|---|---|---|---|
| *Procedure* | **Standard Procedure** | | | **Procedure of the invention** | | | | |
| *Scale* | 0.030 mmol | 0.030 mmol | 0.03 0 mmol | 0.030 mmol | 0.030 mmol | 0.030 mmol | 0.250 mmol | 5.000 mmol |
| Purity of the linear purified precursor | 95% | 95% | 95% | 95% | 95% | 95% | 95% | 95% |
| Yield of the linear purified precursor⁽¹⁾ | 40% | 40% | 40% | 40% | 40% | 40% | 37% | 39% |
| | | | | | | | | |
| **Loop 1 Cyclization method** | O₂ | O₂ | O₂ | O₂ | O₂ | O₂ | O₂ | O₂ |
| Purification method | HPLC | HPLC | HPLC | HPLC | HPLC | HPLC | HPLC | HPLC |
| Purity | 80% | 80% | 80% | 98% | 95% | 97% | 96% | 95% |
| Yield ⁽²⁾ | 35% | 38% | 35% | 27% | 30% | 25% | 28% | 28% |
| | | | | | | | | |
| **Loop 4 Cyclization method** | I₂ | I₂ | I₂ | Tl (trifluoro acetate)₃ | Tl (trifluoro acetate)₃ | Tl (trifluoro acetate)₃ | Tl (trifluoro acetate)₃ | Tl (trifluoro acetate)₃ |
| Purification method | HPLC | HPLC | HPLC | Gel - filtration | Gel- filtration | Gel - filtration | Gel - filtration | Gel - filtration |
| Purity | 90% | 90% | 90% | 96% | 95% | 97% | 95% | 95% |
| Yield ⁽³⁾ | 3% | 2% | 2% | 98% | 100% | 100% | 99% | 99% |
| | | | | | | | | |
| **Overall process yield** | **0.42%** | **0.30%** | **0.28%** | **10.6%** | **12.0%** | **10.0%** | **10.4%** | **10.8%** |

| | | | | | | | | |
|---|---|---|---|---|---|---|---|---|
| (1) - (3) These yields were calculated with respect to the theoretical yield of each step | | | | | | | | |

### EXAMPLE 1 - Small scale synthesis (0.030 mmol) of L1L4 (Ia)

### Solid phase synthesis of the linear precursor (steps a and b)

The linear precursor of L1L4 was synthesized using Fmoc solid phase strategy, using a Fmoc-Cys(Acm)-chlorotrityl resin (substitution 0.4 mmol g⁻¹), to avoid cysteine racernisation. In order to obtain selective formation of the two disulphide bridges (Cys¹/Cys⁸ and Cys¹²/Cys¹⁷) Cys¹ and Cys⁸ were protected with Trt (TFA-labile protecting group) and Cys¹² and Cys¹⁷ were protected with Acm (iodine-labile protecting group).

The linear precursor was synthesised through a series of repeated deprotection/coupling steps. Removal of the Fmoc group was performed with 20% piperidine in DMF. The coupling reactions were performed through activation of the Fmoc-amino acid derivatives with HBTU (4 equivalents) and DIEA (8 equivalents) in DMF. Each coupling was repeated twice and monitored by Kaiser test.

The peptide was cleaved off the resin with contemporary deprotection of all side chain protecting groups (except for Acm on Cys¹² and Cys¹⁷), using a mixture of TFA/H₂O/phenol/thioanisol/EDT/TIS (81.5:5:5:5:2.5:1 ν/ν/ν/ν/ν/ν). The resin was then filtered off and the linear precursor of L1L4 was precipitated from ethyl ether.

The structure of the crude peptide was confirmed by matrix-assisted laser desorption ionization mass spectrometry (MALDI-MS) on a MALDI-TOF Voyager-DE (Perseptive Biosystem) spectrometer. The MALDI-tof analysis revealed a molecular ion peak [M-H⁺] of 1874 as expected for the linear precursor. Analytical HPLC on a SHlMADZU LC-10 system equipped with a Diode Array detector revealed a purity of 60% Elution was performed with a binary system (Eluent A: 0.1%TFA in water; Eluent B: 0.1%TFA in acetonitrile) using a Vydac C₁₈ column (4.6 x 150 mm; 5µm; 300 A) and a linear gradient from 5% B to 65% B in 20 min at a flow rate of 1 ml/min.

The peptide (still protected at Cys¹² and Cys¹⁷ with Acm groups) was obtained in 80% overall yield and was purified by preparative HPLC on a SHIMADZU LC system equipped with a UV detector, eluting with a binary system (Eluent A: 0.1% TFA in water; Eluent B: 0.1% TFA in acetonitrile) using a Vydac C₁₈ column (22 x 250 mm; 5µm; 300 A) and a linear gradient from 5% B to 65% B in 40 min at flow rate of 10 ml/min.

Identity of the purified peptide was confirmed by matrix-assisted laser desorption ionization mass spectrometry (MALDI-MS) on a MALDI-TOF Voyager-DE (Perseptive Biosystem) spectrometer. The MALDI-tof analysis revealed a molecular ion peak [M-H⁺] of 1874 as expected for the linear precursor. Analytical HPLC on a SHIMADZU LC-10 system equipped with a Diode Array detector revealed a purity higher than 95%. Elution was performed with a binary system (Eluent A: 0.1%TFA in water; Eluent B: 0.1%TFA in acetonitrile) using a Vydac C₁₈ column (4.6 x 150 mm; 5µm; 300 A) and a linear gradient from 5% B to 65% B in 20 min at flow rate of 1 ml/min.

The total amount of the recovered pure peptide (purity 95%) was 22.0 mg (40% yield).

### Selective disulphide bridges formation in solution (steps c-f)

The product of step b (0.1 mM) was dissolved in phosphate buffer at pH 7-8 and allowed to stand for 24 h in the presence of atmospheric oxygen to form the Cys¹/Cys⁸ disulphide bridge (loop 1). The reaction progress was monitored by HPLC and when the reaction was complete the product was purified by preparative HPLC under the same conditions as the linear precursor (purity: 97%; yield: 27%).

Formation of the disulphide bridge Cys¹²/Cys¹⁷ was carried out dissolving the product in TFA/anisole (final concentration 0.1 mM) and treating the solution with thallium trifluoroacetate (1.2 equivalents), following the reaction by HPLC. After 60 min the reaction was complete and no side products were detected.

The resulting peptide, containing two disulphide bridges was recovered by gel filtration (yield: 98%; purity: 96%); HPLC was not necessary. The structure was confirmed by MALDI-Tof spectrometry which showed the expected molecular ion peak [M-H⁺] of 1730 Da. 5.5 mg of purified peptide were obtained (10.6% overall yield, 96% purity).

### EXAMPLE 2 - Small scale synthesis (0.030 mmol) of RNL1-L4 (Ib)

The linear precursor and peptide (Ib) were obtained as described in example 1 for L1L4, Peptide (Ib) did not require preparative HPLC, as it was recovered in 95% purity. The structure was confirmed by MALDI-Tof spectrometry which showed the expected molecular ion peak [M-H⁺] of 1742 Da.

6.2 mg of (Ib) was obtained (95% purity; 12% overall yield).

### EXAMPLE 3 - Small scale synthesis (0.030 mmol) of L1-DRL4 (Ic)

The linear precursor and peptide L1-DRL4 (Ic), containig two disulfide bridges, were obtained as described in example 1 for L1L4 5.5 mg of (Ic) were obtained (10.0% overall yield; 97% purity); HPLC was not necessary. The structure was confirmed by MALDI-Tof spectrometry which showed the expected molecular ion peak [M-H⁺] of 1818 Da.

### EXAMPLE 4 - Medium scale synthesis (0.250 mmol) of L1L4

Solid phase synthesis of the linear precursor (steps a and b)

The linear precursor was obtained as described above for small scale synthesis.

The structure of the purified peptide was confirmed through matrix-assisted laser desorption ionization mass spectrometry (MALDI-MS) on a MALDI-TOF Voyager-DE (Perseptive Biosystem) spectrometer. The MALDI-tof analysis gave the molecular ion peak [M-H⁺] of 1874 as expected for the linear precursor. Analytical HPLC on a SHIMADZU LC-10 system equipped with a Diode Array detector showed a purity higher than 95%. Elution was performed with a binary system (Eluent A: 0.1% TFA in water; Eluent B: 0.1% TFA in acetonitrile) using a Vydac C₁₈ column (4.6 x 150 mm; 5µm; 300 A) and a linear gradient from 5% B to 65% B in 20 min at flow rate of 1 ml/min. 187.0 mg of purified peptide was obtained (40% yield; purity 95%).

### Selective disulphide bridges formation in solution (steps c-f)

The product from step b was dissolved in phosphate buffer at pH 7-8 to a final concentration of 0.1 mM and allowed to stand in the presence of atmospheric oxygen for 24 hours, monitoring the formation of the Cys¹/Cys⁸ disulphide bridge (loop 1) by HPLC.

The product was purified by preparative HPLC under the same conditions used for the linear precursor to a final purity of 96% and 28% yield, then dissolved in TFA/anisole to a final concentration of 0.1 mM and added with thallium trifluoroacetate (1.2 equivalents). Removal of the Acm groups and formation of the Cys¹²/Cys¹⁷ disulphide bridge was monitored by HPLC; after 60 min. the reaction was complete and no side products were detected.

The product was recovered by gel filtration (99% yield, 95% purity) and the structure was confirmed by MALDI-Tof spectrometry which gave the expected molecular ion peak [M-H⁺] of 1730 Da. The overall yield was 10.4% (45.0 mg).

### EXAMPLE 5 - Large scale synthesis (5.00 mmol) of L1L4 (Ic)

### Solid phase synthesis of the linear precursor (steps a and b)

The linear precursor was obtained as described above for small scale synthesis and identity was confirmed by matrix-assisted laser desorption ionization mass spectrometry (MALDI-MS) on a MALDI-TOF Voyager-DE (Perseptive Biosystem) spectrometer. The MALDI-tof analysis gave the expected molecular ion peak [M-H⁺] of 1874. Analytical HPLC on the SHIMADZU LC-10 system equipped with a Diode Array detector revealed a purity higher than 95%. Elution was performed with a binary system (Eluent A: 0.1%TFA in water; Eluent B: 0.1°/TFA in acetonitrile) using a Vydac C₁₈ column (4.6 x 150 mm; 5µm; 300 A) and a linear gradient from 5% B to 65% B in 20 min at flow rate of 1 ml/min.

The total amount of purified peptide (purity 95%) was 3.65 g (39% yield).

### Selective disulphide bridges formation in solution (steps c-f)

The product from step b was dissolved in phosphate buffer at pH 7-8 to a final concentration of 0.1 mM and allowed to stand under atmospheric oxygen, monitoring the formation of the disulphide Cys¹/Cys⁸ bridge (loop 1) with HPLC; the reaction was complete after 24 hours.

The product was purified by preparative HPLC under the same conditions as the linear precursor to a final purity of 95% (28% yield), then dissolved in TFA/anisole to a concentration of 0.1 mM and added with thallium trifluoroacetate (1.2 equivalents). Removal of the Acm groups and formation of the Cys¹²/Cys¹⁷ disulphide bridge was monitored by HPLC; after 60 min the reaction was complete and no side products were detected. The product was recovered by gel filtration in 99% yield with 95% purity; and identified by MALDI-Tof spectrometry which gave the expected molecular ion peak [M-H⁺] of 1730 Da. The overall yield was 389.0 mg (10.6%).

## Claims

1. A process for the preparation of cyclic peptides of formula (I): 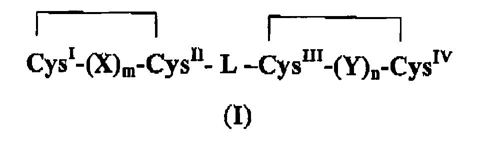 in which:
X and Y, independently from one another, represent each an amino acid;
m and n, independently from one another, represent each an integer ranging from 4 to 8;
L is a linker formed by 2 to 4 amino acids, or an organic linker
the process comprising the following steps:
a) synthesising a linear peptide of formula **(II):**
**Cys¹(P)-(X)ₘ(P)-Cys^{II}(P)- L(P) -Cys^{III}(P)- (Y)ₙ(P) -Cys^{IV}(P)** (II)
wherein X, Y, m, n and L are as defined above and P represent a protecting group, with the proviso that the P groups on Cys^{I} and Cys^{II} are TFA-Iabile protecting groups and the P groups on Cys^{III} and Cys^{IV} are TFA-stable protecting groups;
b) selectively removing the TFA-labile protecting groups with contemporary release of the linear peptide from resin;
c) dissolving the resulting peptide in diluted phosphate buffer at pH 7-8 and exposing the solution to atmospheric oxygen so as to form a disulfide bridge between Cys^{I} and Cys^{II};
d) purifying the resulting peptide by HPLC;
e) dissolving the peptide at a concentration of 0.05 - 0.15 mM in a TFA/anisole solution containing thallium trifluoroacetate so as to form a disulfide bridge between Cys^{III} and Cys^{IV};
f) submitting the resulting peptide to gel filtration.

2. A process according to claim 1 wherein, in the peptide of formula (I), (X)ₘ is an amino acid sequence selected from TDIKGK and TDIRGN.

3. A process according to claim 1 wherein, in the peptide of formula (I), (Y)ₙ is an amino acid sequence selected from DGKQ and DDRQ.

4. A process according to claim 1 wherein, in the peptide of formula (I), L is an organic linker selected from amino carboxylic acids containing a number of methylene units ranging from 2 to 11, or polyoxoethylene amino carboxylic acids containing a number of oxoethylene (-CH2-CH2-O-) units ranging from 2 to 4.

5. A process according to claim 4 wherein, in the peptide of formula (I), L is a TGA sequence.

6. A process according to claim 1 wherein the cyclic peptide is selected from:

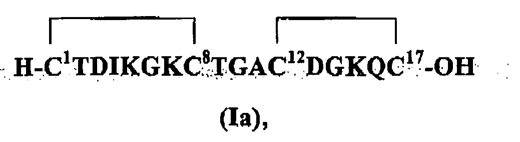

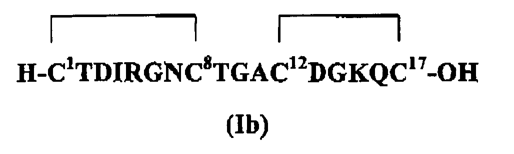

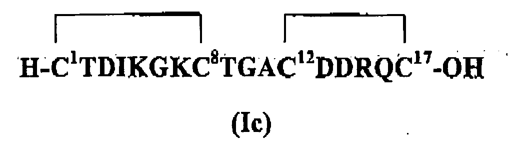

7. A process according to any one of claims 1-6 wherein the TFA-Iabile protecting groups on Cys^{I} and Cys^{II} are trityl groups and the TFA-stable protecting groups on Cys^{III} and Cys^{IV} are acetamidine groups.
